# EUROPEAN PATENT APPLICATION

(11) **EP 1 803 474 A2**
(43) Date of publication of application: **04.07.2007**
(21) Application number: 06256582.5
(22) Date of filing: 27.12.2006
(51) Int. Cl.: A61L 27/34, A61L 27/50, A61L 27/54

(54) **A photoactive biocompatible coating composition**

(30) Priority: 29.12.2005 US 321424
(71) Applicant: Cordis Corporation, Miami Lakes, FL 33014 (US)
(72) Inventor: Falotico, Robert, Belle Mead, NJ 08502 (US); Zhao, Jonathon Z., Belle Mead, NJ 08502 (US)
(74) Representative: Belcher, Simon James

(57) **Abstract**

The present invention discloses a biocompatible coating composition that can be photo crosslinked when exposed to long wavelength ultraviolet light. The biocompatible coating composition comprises at least one biocompatible polymer having a photoactive moiety covalently attached thereto, and a biologically active molecule having a photoactive moiety covalently attached thereto. The photoactive moiety covalently attached to the biologically active molecule and the photoactive moiety covalently attached to the at least one biocompatible polymer are the same or different, and absorb and respond to ultraviolet light having a wavelength band of about 300 nm or above. Preferably, the photoactive moiety is a chemical moiety derived from thioxanthone. The inventive biocompatible coating composition can be applied on at least a portion of one surface of an article.

## Description

The present invention relates to a biocompatible coating composition that can be photo crosslinked when exposed to long wavelength ultraviolet light and an article having the inventive biocompatible coating thereon.

Medical devices, particularly medical implants, are intended for prolonged use and directly interface with body tissues. Thus, the issue of biocompatibility is a critical concern for manufacturers of medical devices. However, most medical devices are made from non-biocompatible materials, such as metals, ceramics, or polymeric materials, which are hydrophobic, non-conformal, and non-slippery. During clinical use or operation, these non-biocompatible materials may cause thrombus formation, inflammation, or other injuries to mucous membranes. In order to function properly and safely, medical devices are usually coated with one or more layers of biocompatible materials. The coatings on these medical devices may, in some instances, be used to deliver therapeutic and pharmaceutical agents.

One requirement for coating materials of medical devices is hemocompatibility since the response of blood to a foreign material can be aggressive, resulting in surface induced thrombus formation, which not only impairs or disables the function of the device, but also threatens patient health. Moreover, it is also required that coating materials of medical devices are mechanically durable so that they do not crack or peel off from the surfaces of the medical devices during use or operation.

To obtain a coating material having both hemocompatibility and mechanical durability, SurModics, Inc. has developed PhotoLink^{®} technology, in which a polymeric coating composition containing photoinitiator-modified polymers and photoinitiator-modified biologically active molecules were crosslinked by exposing the polymeric coating composition to ultraviolet (UV) light. One of the biologically active molecules typically used in PhotoLink^{®} is heparin, a polysaccharide compound that has been used clinically for decades as an intravenous anticoagulant to treat inherent clotting disorders and to prevent blood clot formation during surgery and interventional procedures. When immobilized onto a coating material, heparin improves the hemocompatibility of the coating material. Furthermore, light-activated crosslinking process, i.e., photo crosslinking process, produces covalent chemical bond of the coating to the device surface resulting in a comparatively stable coating.

However, the photoinitiator used in PhotoLink^{®} is a benzophenone or a derivative thereof, which absorb and respond to UV light having a short wavelength band, i.e., UV light having a wavelength band of about 300 nm or less. Thus, the photo crosslinking reaction in PhotoLink^{®} can only be initiated with UV light having a short wavelength band. Notably, many therapeutic agents (e.g., sirolimus) are sensitive to short wavelength UV light and may degrade when exposed to it. In fact, a significant loss of sirolimus content was observed after the photo crosslinking or plasma treatment process. In addition, the long-lasting free radicals generated by the PhotoLink^{®} process may adversely affect the stability of the drugs embedded in the coating, consequently reducing the biological activity of the drugs.

Therefore, there remains a need for a biocompatible coating composition that can undergo a photo crosslinking process with improved retention of the biological activity of the therapeutic agents embedded therein.

Accordingly, the present invention provides a biocompatible coating composition for applying on at least a portion of one surface of an article, comprising at least one biocompatible polymer having a photoactive moiety covalently attached thereto, and a biologically active molecule having a photoactive moiety covalently attached thereto; wherein the photoactive moiety covalently attached to the biologically active molecule and the photoactive moiety covalently attached to the at least one biocompatible polymer are the same or different, and absorb and respond to UV light having a wavelength band of about 300 nm or above. Preferably, the photoactive moiety is a chemical moiety derived from thioxanthone.

The present invention also provides an article having a biocompatible coating thereon, the biocompatible coating comprising at least one biocompatible polymer having a photoactive moiety covalently attached thereto, and a biologically active molecule having a photoactive moiety covalently attached thereto; wherein the photoactive moiety covalently attached to the biologically active molecule and the photoactive moiety covalently attached to the at least one biocompatible polymer are the same or different, and absorb and respond to UV light having a wavelength band of about 300 nm or above. Preferably, the article is a medical device or a component of a medical device.

The present invention provides a biocompatible coating composition for applying on at least a portion of one surface of an article. The term "biocompatible" as used herein denotes being biologically compatible by not producing a toxic, injurious, or immunological response in living tissue. The biocompatible coating composition comprises at least one biocompatible polymer having a photoactive moiety covalently attached thereto, and a biologically active molecule having a photoactive moiety covalently attached thereto. The term "biologically active molecule" as used herein denotes a compound or substance having an effect on or eliciting a response from living tissue. The photoactive moiety covalently attached to the biologically active molecule and the photoactive moiety covalently attached to the at least one biocompatible polymer are the same or different, and absorb and respond to ultraviolet light having a wavelength band of about 300 nm or above. In addition to the at least one biocompatible polymer having a photoactive moiety covalently attached thereto and the biologically active molecule having a photoactive moiety covalently attached thereto, the inventive biocompatible coating composition may optionally further comprise one or more biocompatible polymers that do not have any photoactive moiety covalently attached thereto.

The photoactive moieties of the present invention absorb UV light having a long wavelength band, i.e., a wavelength band of about 300 nm or above, and consequently initiate a photo crosslinking reaction. It is preferred that the photoactive moieties of the present invention have an extinction coefficient higher than that of benzophenone, so that the light exposure time necessary to initiate crosslinking reactions is shortened.

In one embodiment of the present invention, the photoactive moieties are chemical moieties derived from thioxanthone. Typically, the photoactive moieties derived from thioxanthone have the following structure: 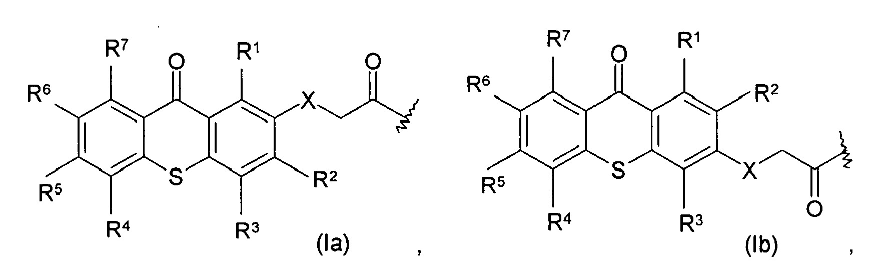 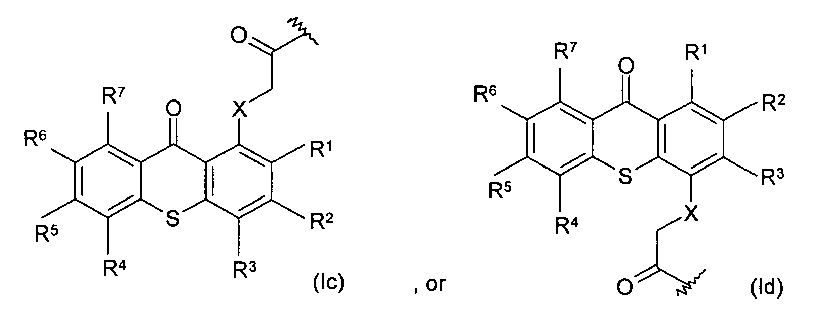 wherein R¹, R², R³, R⁴, R⁵, R⁶, and R⁷ are the same or different, and are hydrogen, halogen, hydroxy, amino, -CN, -COOH, -NO₂, phenylsulfonyl, C1-C12 alkyl, C3-C6 cycloalkyl, C1-C12 alkoxy, C1-C12 alkylthio, -NHR⁸, or N,N-dialkylamino having 1 to 6 carbon atoms in the alkyl moiety; X is CH₂, O, S, NR⁸; and R⁸ is C1-C6 alkyl. Preferably, R¹, R², R³, R⁴, R⁵, R⁶, and R⁷ are independently hydrogen, halogen, hydroxy, or C1-C12 alkyl. Examples of alkyl suitable for the present invention include, but are not limited to: methyl, ethyl, n-propyl, isopropyl, *n*-butyl, *n*-pentyl, *n*-hexyl, and analogs thereof. The photoactive moieties of formula (Ia), (Ib), (Ic), and (Id) not only absorb and respond to UV light having a wavelength of about 300 nm and above, but also have an extinction coefficient higher than that of benzophenone.

It is preferred that the at least one biocompatible polymer having a photoactive moiety covalently attached thereto is derived from conjugation of a biocompatible polymer or an analog thereof and a reactive photoinitiator. The reactive photoinitiator comprises a photoactive moiety and at least one functional group, wherein the photoactive moiety absorbs and responds to UV light having a wavelength band of about 300 nm or above, and the at least one functional group reacts with the biocompatible polymer or an analog thereof forming a covalent bond. The biocompatible polymers suitable for the present invention include, but are not limited to: poly(ethylene glycol), poly(vinyl pyrrolidone), poly(alkyl acrylate), and poly(alkyl methacrylate). Preferably, the biocompatible polymer is a water-soluble polymer. In one embodiment of the present invention, the water-soluble polymer is poly(vinyl pyrrolidone). The term "photoinitiator" denotes an organic compound that absorbs and responds to ultraviolet light and produces free radicals or cations initiating a cross-linking polymerization process. The functional group in the reactive photoinitiator of the present invention may be any nucleophilic or electrophilic chemical moiety that can react with the biocompatible polymer to form a covalent bond. Examples of the functional group suitable for the reactive photoinitiator in the present invention include, but are not limited to: carboxylic acid, carboxylic acid chloride, amino, hydroxy, halogen-substituted alkyl, thiocarboxyl, and triazole.

In one embodiment of the present invention, the reactive photoinitiator is a thioxanthone-type photoinitiator having a structure as follows: 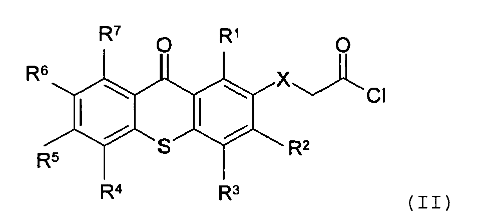 wherein R¹, R², R³, R⁴, R⁵, R⁶, and R⁷are the same or different, and are hydrogen, halogen, hydroxy, amino, -CN, -COOH, -NO₂, phenylsulfonyl, C1-C12 alkyl, C3-C6 cycloalkyl, C1-C12 alkoxy, C1-C12 alkylthio, -NHR⁸, or N,N-dialkylamino having 1 to 6 carbon atoms in the alkyl moiety; X is CH₂, O, S, NR⁸; and R⁸ is C1-C6 alkyl. Preferably, R¹, R², R³, R⁴, R⁵, R⁶, and R⁷ are independently hydrogen, halogen, hydroxy, or C1-C12 alkyl.

The synthesis of thioxanthone-type photoinitiators has bee reported in the literature and is well known to one skilled in the art. A typical synthetic scheme for preparing thioxanthone-type photoinitiators is illustrated in Scheme 1, wherein X is CH₂, O, S, NR⁸; and R⁸ is C1-C6 alkyl.

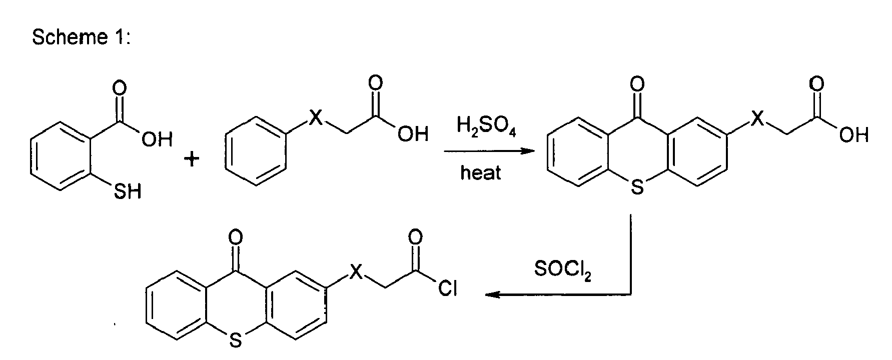

In one embodiment of the present invention, the at least one biocompatible polymer having a photoactive moiety covalently attached thereto is derived from poly(vinyl pyrrolidone) and a reactive photoinitiator, and has the following structure: 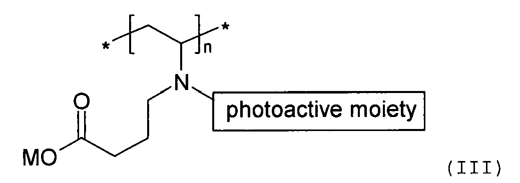 wherein M is an alkali metal cation, n is an integer of 10 to 5000. Preferably, M is sodium cation or potassium cation.

In another embodiment of the present invention, as illustrated in Scheme 2, the at least one biocompatible polymer having a photoactive moiety covalently attached thereto is derived from poly(vinyl pyrrolidone) and a thioxanthone-type photoinitiator of formula (II). The "base" as used herein may be any base that can hydrolize a lactamide. Preferably, the base is an alkali hydroxide, such as sodium hydroxide and potassium hydroxide. 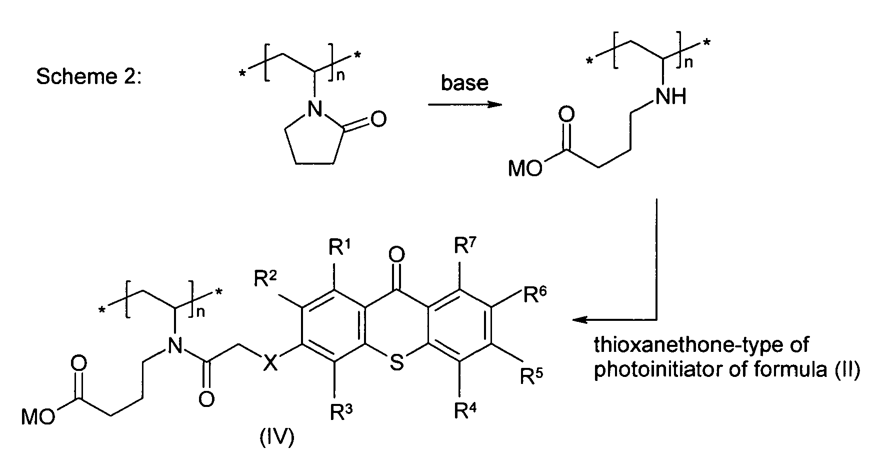 wherein R¹, R², R³, R⁴, R⁵, R⁶, and R⁷ are the same or different, and are hydrogen, halogen, hydroxy, amino, -CN, -COOH, -NO₂, phenylsulfonyl, C1-C12 alkyl, C3-C6 cycloalkyl, C1-C12 alkoxy, C1-C12 alkylthio, -NHR⁸, or N,N-dialkylamino having 1 to 6 carbon atoms in the alkyl moiety; X is CH₂, O, S, NR⁸; R⁸ is C1-C6 alkyl; M is an alkali metal cation; and n is an integer of 10 to 5000.

In another embodiment of the present invention, as illustrated in Scheme 3, the at least one biocompatible polymer having a photoactive moiety covalently attached thereto is derived from a di-functional reactive photoinitiator and two biocompatible polymers. The term "a di-functional reactive photoinitiator" as used herein denotes a reactive photoinitiator comprising a photoactive moiety and two functional groups, wherein the photoactive moiety absorbs and responds to UV light having a wavelength band of about 300 nm or above, and the two functional groups react with two biocompatible polymers or analogs thereof independently forming two covalent bonds. The inventive biocompatible coating composition comprising the at least one biocompatible polymer having a photoactive moiety covalently attached thereto that is derived from a di-functional reactive photoinitiator has a further enhanced integrity and stability. Polymer¹ and Polymer² in Scheme 3 are the same or different, and are both biocompatible polymers. Preferably, Polymer¹-NH₂ and Polymer²-NH₂ are copolymers of poly(butyl methacrylate) and poly(methyl methacrylate) with amine end groups.

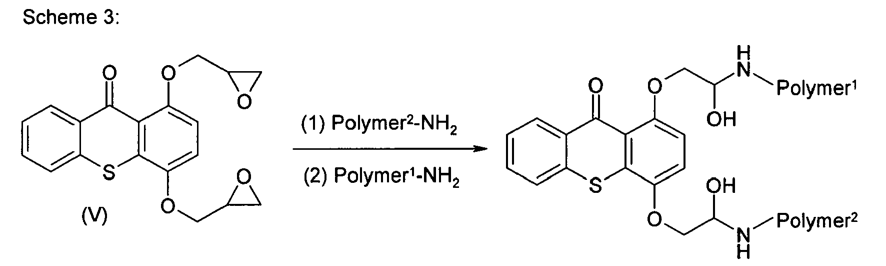

It is preferred that the biologically active molecule having a photoactive moiety covalently attached thereto is derived from conjugation of a biologically active molecule or an analog thereof and a reactive photoinitiator. The reactive photoinitiator comprises a photoactive moiety and at least one functional group, wherein the photoactive moiety absorbs and responds to ultraviolet light having a wavelength band of about 300 nm or above, and the at least one functional group reacts with the biologically active molecule or an analog thereof forming a covalent bond. In one embodiment of the present invention, the reactive photoinitiator has a structure of formula (II), as defined hereinabove.

The biologically active molecules suitable for the present invention include, for example, any drugs, agents, compounds and/or combination thereof that have therapeutic effects for treating or preventing a disease or a biological organism's reaction to the introduction of the medical device to the organism. Preferred biological active molecules include, but are not limited to: anti-thrombogenic agents, immuno-suppressants, anti-neoplastic agents, anti-inflammatory agents, angiogenesis inhibitors, protein kinase inhibitors, and other agents which may cure, reduce, or prevent restenosis in a mammal. Examples of the biological active molecules of the present invention include, but are not limited to: heparin, albumin, streptokinase, tissue plasminogin activator (TPA), urokinase, rapamycin, paclitaxel, pimecrolimus, and their analogs and derivatives.

In one embodiment of the present invention, as illustrated in Scheme 4, the biologically active molecule having a photoactive moiety attached thereto is prepared from a heparin analog and a thioxanthone-type photoinitiator of formula (II). 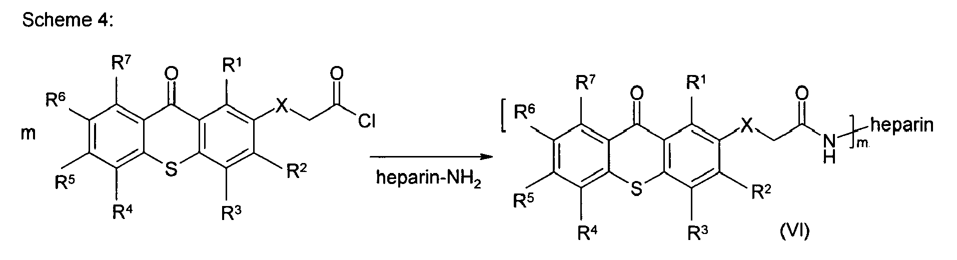 wherein m is an integer of 1 to 20; and R¹, R², R³, R⁴, R⁵, R⁶, and R⁷ are as defined hereinabove.

In the present invention, the at least one biocompatible polymer having a photoactive moiety attached thereto and the biologically active molecule having a photoactive moiety attached thereto are prepared separately, and then are physically admixed together in any manner known to one skilled in the art to form the inventive biocompatible coating composition. In one embodiment of the present invention, the inventive biologically active molecule having a photoactive moiety attached thereto is dissolved in an organic solvent, and the resulting solution is then admixed with a solution of the at least one biocompatible polymer having a photoactive moiety attached thereto. In another embodiment of the present invention, the biologically active molecule having a photoactive moiety attached thereto is directly dissolved in a solution of the at least one biocompatible polymer having a photoactive moiety attached thereto in an organic solvent.
Depending on the intended use of the inventive biocompatible coating composition, one or more biocompatible polymers that do not have any photoactive moiety covalently attached thereto may optionally be admixed with the separately prepared at least one biocompatible polymer having a photoactive moiety attached thereto and the biologically active molecule having a photoactive moiety attached thereto.

When exposed to UV light having a wavelength band of about 300 nm or above, the inventive biocompatible coating composition undergoes a crosslinking reaction so that the at least one biocompatible polymer having a photoactive moiety covalently attached thereto and the biologically active molecule having a photoactive moiety covalently attached thereto are crosslinked. Since the light source used in the photo crosslinking process of the present invention is UV light having a long wavelength band, the damage to the biologically active molecule within the inventive biocompatible coating composition is significantly reduced comparing to the damage to the biologically active molecule in the PhotoLink^{®} process. Furthermore, the inventive biocompatible coating composition has an extinction coefficient higher than that of benzophenone, consequently, the exposure time required for initiating a photo cross-linking process in the present invention is substantially shortened. Thus, the damage to the biologically active molecule is further reduced. That is, unlike the prior art Photolink^{®} technology, the inventive biocompatible coating composition can undergo a photo crosslinking process causing no damage or little damage to the biologically active molecule embedded therein. Therefore, the biologically active molecule within the inventive biocompatible composition retains improved biological activity comparing to the biologically active molecule in the prior art Photolink^{®} technology.

The physiomechanical properties (e.g., smoothness, lubricity, and durability) of the inventive biocompatible composition may be tuned by varying the biocompatible polymers used for conjugation with a reactive photoinitiator or by introducing one or more biocompatible polymers that do not have any photoactive moiety covalently attached thereto. Furthermore, various functional groups may be introduced into the photoactive moieties of the present invention to impart desirable physiochemical properties to the at least one biocompatible polymer or the biological molecule. The optimal photo crosslinking condition of the present invention may be obtained by adjusting the density of the photoactive moieties in the inventive biocompatible coating composition, deploying appropriate lighting sources, or a combination thereof. The density of the photoactive moieties in the biocompatible coating composition may be controlled by adjusting the feed ratio of the reactive photoinitiator to the biocompatible polymer or the biologically active molecule. Lighting sources suitable for the present invention include, but are not limited to: LED lights with long wavelength at around 400 nm and mercury lamps doped with metal halides that have maximal absorption at UV light having a wavelength band longer than about 300 nm.

The inventive biocompatible coating composition may be applied to at least a portion of one surface of an article. In some embodiments, the inventive biocompatible coating composition is applied to all exposed surfaces of an article. The thickness of the biocompatible coating may vary depending on the process used in forming the coating as well as the intended use of the article. Typically, and for a medical device, the inventive coating is applied to a thickness from about 1 nanometer to about 10 micrometers, with a thickness from about 100 nanometers to about 10 micrometers being more typical.

The present invention also provides an article having the inventive biocompatible coating thereon. The inventive biocompatible coating is on at least a portion of one surface of the article. The at least a portion of one surface of the article may be a surface of a polymeric coat, a plastic substance, ceramic, steel, or other alloy metals. The article that may be coated with the inventive biocompatible coating composition may be in any shape, and is preferably a medical device or a component of a medical device. The term "medical device" as used herein denotes a physical item used in medical treatment, which includes both external medical devices and implantable medical devices. The medical devices that may be coated with the inventive biocompatible coating material include, but are not limited to: catheters, guidewires, drug eluting stents, cochlear implants, retinal implants, gastric bands, neurostimulation devices, muscular stimulation devices, implantable drug delivery devices, intraocular devices, and various other medical devices. Preferably, the medical device is an implantable medical device, i.e., a medical implant.

The present biocompatible coating material may be applied to the surface of an article using conventional coating techniques, such as, for example, spray coating, ultrasonic coating, dip coating, and the like. In a dip coating process, the article is immersed in a bath containing the biocompatible coating material and then removed. A dwelling time ranging from about 1 minute to about 2 hours may be used depending of the material of construction, complexity of the device, and the desired coating thickness. Next, the article coated with the biocompatible coating material may be allowed to dry to provide a dry coating. Drying may be accomplished merely by standing at ambient conditions or may be accelerated by heating at mild temperatures, such as about 30°C to about 65°C.

## Claims

1. A biocompatible coating composition for applying on at least a portion of one surface of an article, comprising at least one biocompatible polymer having a photoactive moiety covalently attached thereto, and a biologically active molecule having a photoactive moiety covalently attached thereto; wherein the photoactive moiety covalently attached to the biologically active molecule and the photoactive moiety covalently attached to the at least one biocompatible polymer are the same or different, and absorb and respond to ultraviolet light having a wavelength band of about 300 nm or above.

2. The biocompatible coating composition of claim 1, wherein the photoactive moieties are chemical moieties having the following structure: 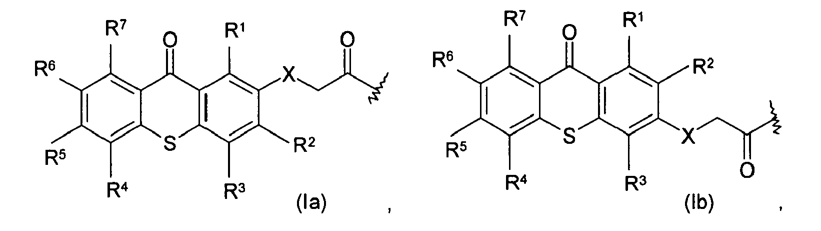 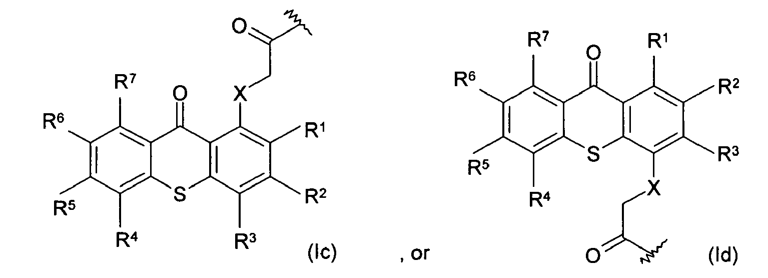 wherein R¹, R², R³, R⁴, R⁵, R⁶, and R⁷ are the same or different, and are hydrogen, halogen, hydroxy, amino, -CN, -COOH, -NO₂ phenylsulfonyl, C1-C12 alkyl, C3-C6 cycloalkyl, C1-C12 alkoxy, C1-C12 alkylthio, -NHR⁸, or N,N-dialkylamino having 1 to 6 carbon atoms in the alkyl moiety; X is CH₂, O, S, NR⁸; and R⁸ is C1-C6 alkyl.

3. The biocompatible coating composition of claim 1, wherein the at least one biocompatible polymer having a photoactive moiety covalently attached thereto is derived from a biocompatible polymer and a reactive photoinitiator, wherein the reactive photoinitiator comprises a photoactive moiety and a functional group, wherein the photoactive moiety absorbs and responds to ultraviolet light having a wavelength band of about 300 nm or above, and the functional group reacts with the biocompatible polymer forming a covalent bond.

4. The biocompatible coating composition of claim 3, wherein the biocompatible polymer is a water-soluble polymer.

5. The biocompatible coating composition of claim 1, wherein the at least one biocompatible polymer having a photoactive moiety covalently attached thereto comprising a repeating unit having the following structure: 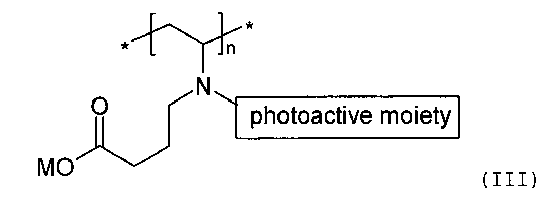 wherein M is an alkali metal cation, n is an integer of 10 to 5000.

6. The biocompatible coating composition of claim 1, wherein the at least one biocompatible polymer having a photoactive moiety covalently attached thereto comprising a repeating unit having the following structure: 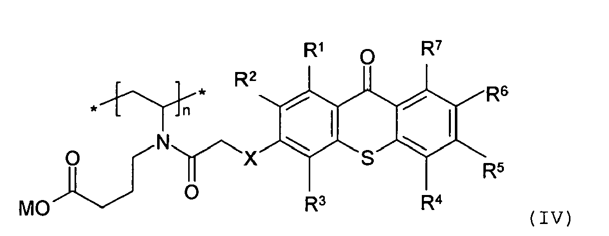 wherein R¹, R², R³, R⁴, R⁵, R⁶, and R⁷ are the same or different, and are hydrogen, halogen, hydroxy, amino, -CN, -COOH, -NO₂, phenylsulfonyl, C1-C12 alkyl, C3-C6 cycloalkyl, C1-C12 alkoxy, C1-C12 alkylthio, -NHR⁸, or N,N-dialkylamino having 1 to 6 carbon atoms in the alkyl moiety; X is CH₂, O, S, NR⁸; M is an alkali metal cation; n is an integer of 10 to 5000; and R⁸ is C1-C6 alkyl.

7. The biocompatible coating composition of claim 1, wherein the biologically active molecule having a photoactive moiety covalently attached thereto is derived from a biologically active molecule and a reactive photoinitiator, wherein the reactive photoinitiator comprises a photoactive moiety and a functional group, wherein the photoactive moiety absorbs and responds to ultraviolet light having a wavelength band of about 300 nm or above, and the functional group reacts with the biologically active molecule forming a covalent bond.

8. The biocompatible coating composition of claim 7, wherein the biologically active molecule is selected from the group consisting of anti-thrombogenic agents, immuno-suppressants, anti-neoplastic agents, anti-inflammatory agents, angiogenesis inhibitors, and protein kinase inhibitors.

9. The biocompatible coating composition of claim 7, wherein the biologically active molecule is an anti-thrombogenic agent, or an immuno-suppressant.

10. The biocompatible coating composition of claim 7, wherein the biologically active molecule is heparin, or sirolimus.

11. The biocompatible coating composition of claim 3 or 7, wherein the reactive photoinitiator has the following structure: 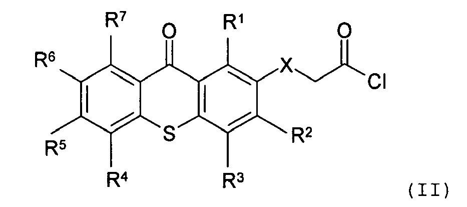 wherein R¹, R², R³, R⁴, R⁵, R⁶, and R⁷ are the same or different, and are hydrogen, halogen, hydroxy, amino, -CN, -COOH, -NO₂, phenylsulfonyl, C1-C12 alkyl, C3-C6 cycloalkyl, C1-C12 alkoxy, C1-C12 alkylthio, -NHR⁸, or N,N-dialkylamino having 1 to 6 carbon atoms in the alkyl moiety; X is CH₂, O, S, NR⁸; and R⁸ is C1-C6 alkyl.

12. The biocompatible coating composition of claim 3 or 7, wherein the reactive photoinitiator has the following structure:

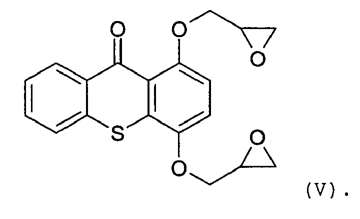

13. The biocompatible coating composition of claim 1, wherein the biologically active molecule having a photoactive moiety covalently attached thereto has the following structure: 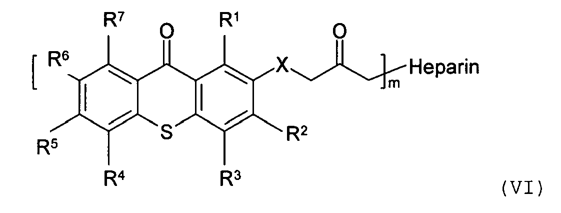 wherein R¹, R², R³, R⁴, R⁵, R⁶, and R⁷ are the same or different, and are hydrogen, halogen, hydroxy, amino, -CN, -COOH, -NO₂, phenylsulfonyl, C1-C12 alkyl, C3-C6 cycloalkyl, C1-C12 alkoxy, C1-C12 alkylthio, -NHR⁸, or N,N-dialkylamino having 1 to 6 carbon atoms in the alkyl moiety; X is CH₂, O, S, NR⁸; m is an integer of 10 to 5000; R⁸ is C1-C6 alkyl; and m is an integer of 1 and 20.

14. The biocompatible coating composition of claim 1, wherein the coating composition has a thickness of about 1 nanometer to about 10 micrometer.

15. An article having a biocompatible coating thereon, the biocompatible coating comprising at least one biocompatible polymer having a photoactive moiety covalently attached thereto, and a biologically active molecule having a photoactive moiety covalently attached thereto; wherein the photoactive moiety covalently attached to the biologically active molecule and the photoactive moiety covalently attached to the at least one biocompatible polymer are the same or different, and absorb and respond to ultraviolet light having a wavelength band of about 300 nm or above.

16. The article of claim 15, wherein the article is a medical device or a component of a medical device.

17. The article of claim 16, wherein the medical device or the component of a medical device is implantable.

18. The article of claim 15, wherein the photoactive moieties are chemical moieties having the following structure: 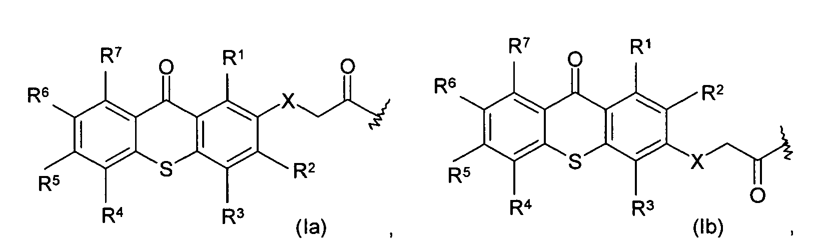 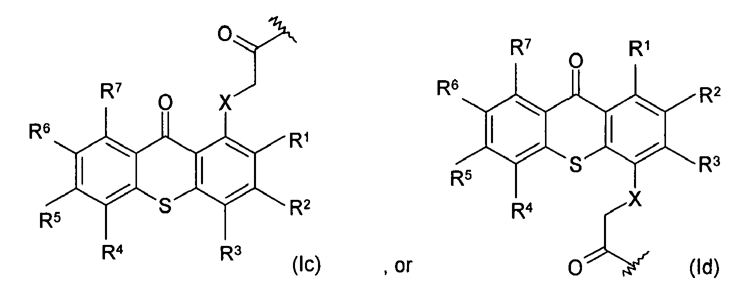 wherein R¹, R², R³, R⁴, R⁵, R⁶, and R⁷ are the same or different, and are hydrogen, halogen, hydroxy, amino, -CN, -COOH, -NO₂, phenylsulfonyl, C1-C12 alkyl, C3-C6 cycloalkyl, C1-C12 alkoxy, C1-C12 alkylthio, -NHR⁸, or N,N-dialkylamino having 1 to 6 carbon atoms in the alkyl moiety; X is CH₂, O, S, NR⁸; and R⁸ is C1-C6 alkyl.

19. The article of claim 15, wherein the at least one biocompatible polymer having a photoactive moiety covalently attached thereto comprising a repeating unit having the following structure: 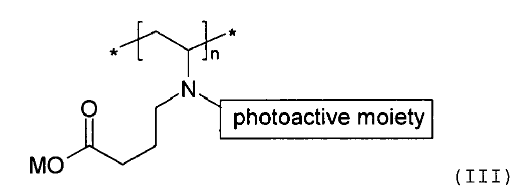 wherein M is an alkali metal cation, n is an integer of 10 to 5000.

20. The article of claim 15, wherein the at least one biocompatible polymer having a photoactive moiety covalently attached thereto comprising a repeating unit having the following structure: 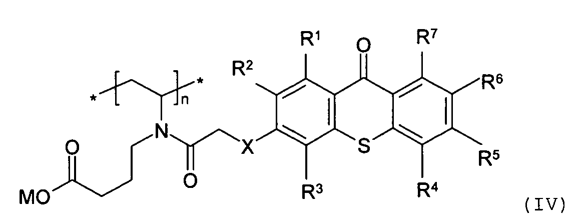 wherein R¹, R², R³, R⁴, R⁵, R⁶, and R⁷ are the same or different, and are hydrogen, halogen, hydroxy, amino, -CN, -COOH, -NO₂, phenylsulfonyl, C1-C12 alkyl, C3-C6 cycloalkyl, C1-C12 alkoxy, C1-C12 alkylthio, -NHR⁸, or N,N-dialkylamino having 1 to 6 carbon atoms in the alkyl moiety; X is CH₂, O, S, NR⁸; M is an alkali metal cation; n is an integer of 10 to 5000; and R⁸ is C1-C6 alkyl.

21. The article of claim 15, wherein the biologically active molecule having a photoactive moiety covalently attached thereto has the following structure: 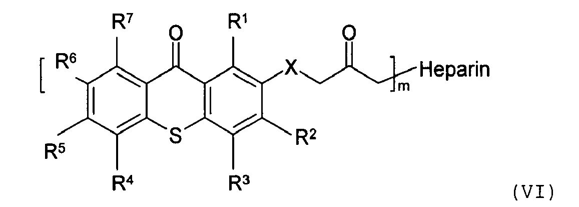 wherein R¹, R², R³, R⁴, R⁵, R⁶, and R⁷ are the same or different, and are hydrogen, halogen, hydroxy, amino, -CN, -COOH, -NO₂, phenylsulfonyl, C1-C12 alkyl, C3-C6 cycloalkyl, C1-C12 alkoxy, C1-C12 alkylthio, -NHR⁸, or N,N-dialkylamino having 1 to 6 carbon atoms in the alkyl moiety; X is CH₂, O, S, NR⁸; m is an integer of 10 to 5000; R⁸ is C1-C6 alkyl; and m is an integer of 1 and 20.

22. The article of claim 15, wherein the coating composition has a thickness of about 1 nanometer to about 10 micrometers.
